# EUROPEAN PATENT SPECIFICATION

(11) **EP 3 280 535 B1**
(45) Date of publication and mention of the grant of the patent: **23.06.2021**
(21) Application number: 16717766.6
(22) Date of filing: 07.04.2016
(51) Int. Cl.: B03C 1/01, B03C 1/033, B03C 1/28, A61M 1/36, C12M 1/00

(54) **DE-BEADING**
ENTFERNUNG VON BEADS
SUPPRESSION DE BULLES

(30) Priority: 07.04.2015 US 201562144226 P
(43) Date of publication of application: 14.02.2018
(73) Proprietor: Terumo BCT, Inc., Lakewood, CO 80215 (US)
(72) Inventor: LADTKOW, Tanner J., Arvada, Colorado 80005 (US); ELLINGBOE, Bruce, Littleton, Colorado 80120 (US); SCHOLLER, Jennifer D., Louisville, Colorado 80027 (US); PITTINGER, John J., Evergreen, Colorado 80439 (US)
(74) Representative: J A Kemp LLP
(86) International application number: PCT/US2016/026517
(87) International publication number: WO 2016/164635

(56) References cited:
- EP-A2- 0 672 458
- WO-A2-01/83002
- US-A- 4 710 472
- US-A- 4 910 148
- US-A- 5 536 475
- US-A- 6 036 857
- US-A1- 2010 297 733
- US-A1- 2011 117 577
- US-A1- 2014 333 453

## Description

### Field

The present invention relates to a system for removing magnetic beads from a fluid stream comprising cells.

### Background

Beads are used in a variety of different applications including in biological applications, such as the growing of cells or other organisms. In some processes, cells may be grown, functionalized, or activated for therapeutic purposes using beads. After the cells have been grown, functionalized, or activated, the beads must be separated from the cells before the cells can be further used.

When separating beads from cells, it is desirable to remove as many of the beads as possible, while not losing cells. In some processes of removing beads, a large number of cells may be lost.

US 5,536,475 discloses an apparatus for magnetic cell separation using paramagnetic microbeads which includes a base upon which is movably carried a rocker member. The rocker member carries both a primary processing container in which a mixture of liquid with cells and paramagnetic microbeads is received; and a primary magnet movable from a first position adjacent to the primary container to magnetically capture the microbeads, and a second position spaced from the primary container to magnetically release the microbeads. The mixture of liquid with cells and microbeads is agitated by tilting motions of the rocker assembly, thereby achieving the binding of target cells to microbeads. The paramagnetic microbeads, with bound target cells, are captured and released by movements of the primary magnet between its first and second positions. A secondary magnet is placed downstream from the primary magnet to capture any paramagnetic microbeads which might escape from the primary magnet. Also provided is an interconnected container apparatus for use to contain and appropriately conduct the liquid, cells, and microbeads.

Embodiments of the present invention have been made in light of these and other considerations. However, the relatively specific problems discussed above do not limit the applicability of the embodiments of the present invention.

### Summary

The summary is provided to introduce aspects of some embodiments of the present invention in a simplified form, and is not intended to identify key or essential elements of the claimed invention, nor is it intended to limit the scope of the claims.

The invention is defined in the claims. Embodiments relating to the method of removing magnetic beads from a fluid stream comprising cells are not covered by the claimed invention.

Some embodiments relate to systems and methods for separating cells from beads. In embodiments, the separation may involve the use of one or more separation chambers that utilize centrifugal forces to separate the beads. Embodiments may provide for use of magnetic fields and flow cells to remove beads that may include ferromagnetic materials (e.g., iron, cobalt, nickel, rare earth metals, etc.).

Other embodiments provide for systems and methods of removing magnetic beads (e.g., which may contain iron, cobalt, nickel, rare earth metals etc.) from a fluid stream comprising cells. Embodiments utilize a fluid pathway through which the fluid stream flows. The fluid pathway includes an indirect pathway in a bag. A magnetic field is provided adjacent the bag, and a pump is used to move the fluid through the bag. As the fluid flows through the indirect pathway, the magnetic field attracts the magnetic beads toward a wall of the bag. By maintaining the beads against the wall of the bag, the magnetic beads are removed from the fluid stream.

In some embodiments, the magnetic field may be generated by a number of magnets in an array. The array may be arranged in a predetermined way (with magnetic poles in particular positions) to control the magnetic field. In embodiments, the magnetic field may be strong enough to maintain the magnetic beads against a wall of the bag.

Additionally, the indirect flow path of the bag may be provided by a variety of features. For example, the indirect flow path may be provided by a channel in a serpentine bag. In other examples, the bag may include barriers around which the fluid stream flows, which creates the indirect flow path. In embodiments, the indirect pathway of the bag provides a long enough flow path to allow the magnetic field to remove at least about ninety percent of the magnetic beads from the fluid stream.

In some embodiments, an outlet of the bag may be connected to components that additionally process the fluid stream. For example, the outlet of the bag may be fluidly connected to a chamber for concentrating the cells in the fluid stream after the magnetic beads have been removed from the fluid stream. As part of the concentration of cells, the chamber may remove liquid and also remaining beads from the fluid stream.

### Brief Description of the Drawings

Non-limiting and non-exhaustive embodiments are described with reference to the following figures.
FIG. 1 illustrates an embodiment of a system that may be utilized for processing a fluid stream with beads and cells according to embodiments.
FIG. 2 illustrates a fluid conveyance assembly that may be utilized in embodiments with a system such as for example the system shown in FIG. 1.
FIG. 3 illustrates an embodiment of a separation chamber system utilizing two chambers that may be used to concentrate cells and/or remove beads according to embodiments.
FIG. 4 illustrates a separation chamber that may be used to concentrate cells and/or remove beads from a fluid stream.
FIG. 5 illustrates a close-up view of a separation chamber with some beads collected at a bottom port/tubing.
FIG. 6 illustrates a cross-sectional view of another embodiment of a separation chamber that may be used to concentrate cells and/or separate beads from cells.
FIG. 7 illustrates a system for processing a fluid stream that may include beads and cells.
FIG. 8 illustrates a top view of a flow cell that may be used to remove magnetic beads according to an embodiment not covered by the claimed invention.
FIG. 9 illustrates an exploded view of the flow cell of FIG. 8.
FIG. 10 illustrates a cross-sectional (partially exploded) view of the flow cell of FIG. 8.
FIGS. 11A-E illustrate different views of another flow cell that is used to remove magnetic beads according to an embodiment of the invention.
FIGS. 12A-12F illustrate different designs of bags with indirect paths that may be used in some flow cells according to embodiments.
FIG. 13A-E illustrates embodiments of magnet arrays that may be used in some embodiments of flow cells.
FIG. 14 illustrates a flow chart of a process for removing magnetic beads from a fluid stream according to an embodiment not covered by the claimed invention.

### Detailed Description

The principles of the present invention may be further understood by reference to the following detailed description and the embodiments depicted in the accompanying drawings. It should be understood that although specific features are shown and described below with respect to detailed embodiments, the present invention is not limited to the embodiments described below.

Reference will now be made in detail to the embodiments illustrated in the accompanying drawings and described below. Wherever possible, the same reference numbers are used in the drawings and the description to refer to the same or similar parts.

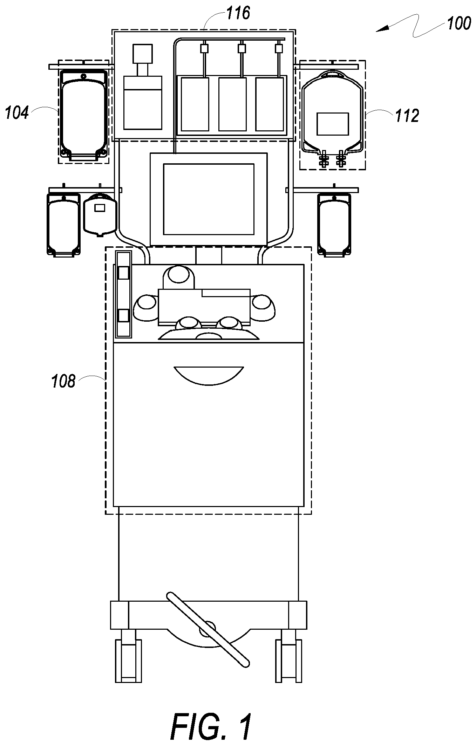

FIG. 1 illustrates an embodiment of a system 100 that may be utilized for processing a fluid stream that may include beads and cells. In some embodiments, the cells may be human T cells and the beads may be made from a magnetic material with a polymeric coating. Some embodiments of system 100 may provide for separating (e.g., removing) beads from the stream of fluid that includes the cells. In addition, system 100 may also be used to concentrate, wash, and add a storage solution to cells. FIG. 1 illustrates a storage bag 104 that in embodiments stores a fluid that includes cells and beads utilized for growing, functionalizing, and/or activating cells. In order for the cells to be used for therapeutic purposes, the cells may be separated from the beads and concentrated to provide for a usable/effective dose.

Separation/concentration system 108 may be part of system 100 and may be used to de-bead (e.g., remove beads from) a fluid stream that includes cells, and also may be used to concentrate the cells. As described in greater detail below, the separation/concentration system 108 may utilize a variety of systems, components, fluid flow paths, etc. to separate beads from a fluid stream and/or concentrate cells after separation of beads from the fluid stream.

As part of the fluid processing, some embodiments provide for washing the cells using the separation/concentration system 108 in addition to separating beads and concentrating cells. A wash solution 112 may be used by the separation/concentration system 108 to wash the cells. Examples of systems and methods that may be used as part of system 108 to concentrate and wash cells are described in U.S. Patent Publication No. US 2006/0045921 A1, entitled "PROCESSING PARTICLES".

After the cells are de-beaded, concentrated, and washed, a fill system 116 may separate the concentrated/washed cells into separate storage containers each with a predetermined concentration and/or volume of cells. In one embodiment, fill system 116 may fill each storage container with what may be considered one dose of therapeutic cells. In addition, fill system 116 may also add a storage solution to the individual storage containers. As one example, a predetermined amount of DMSO may be added by fill system 116 to each individual storage container.

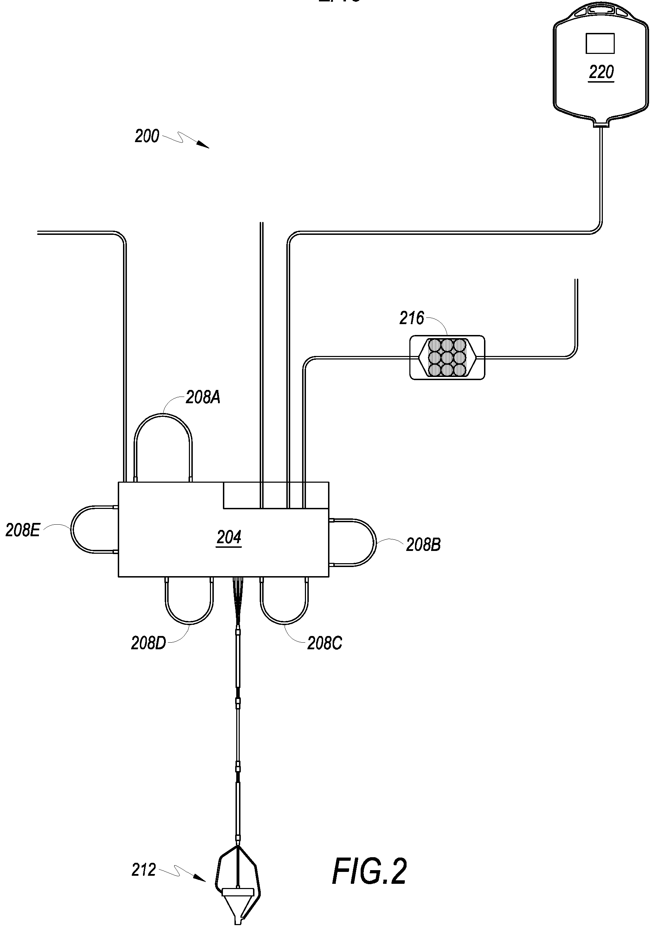

FIG. 2 illustrates a fluid conveyance assembly 200 that may be utilized in embodiments with the system shown in FIG. 1. In embodiments, assembly 200 may be a disposable component that is utilized once in a batch process that separates beads from cells, concentrates the cells, washes the cells, and stores the cells in storage containers. In other embodiments, fluid conveyance assembly 200 may be reposable, so that it is reused, after for example a sterilization process.

As shown in FIG. 2, assembly 200 includes a cassette 204, which routes tubing that is connected to various bags and/or other components. Cassette 204 also includes a variety of tubing loops 208A-E which may engage pumps for pumping fluid to and from different locations or components, e.g., separation/concentration system 108, fill system 116 (system 100), separation chamber 212, flow cell 216, waste bag 220, etc.

As noted above, assembly 200 also includes separation chamber 212 where cells and beads may be separated in some embodiments. In other embodiments, chamber 212 may be used simply to concentrate and wash cells after beads have been separated from the cells, as described in greater detail below.

In the embodiment shown in FIG. 2, a flow cell 216 is also provided to separate beads from cells. As described in greater detail below, flow cells (e.g., 216) may be magnetic flow cells that utilize a magnetic field to separate beads from cells.

As shown in FIG. 2, assembly 200 also includes a waste bag 220, into which fluid or other materials may be transferred. In embodiments, waste bag 220 may store used wash fluid as well as fluid removed from a fluid stream that includes cells, in order to concentrate the cells.

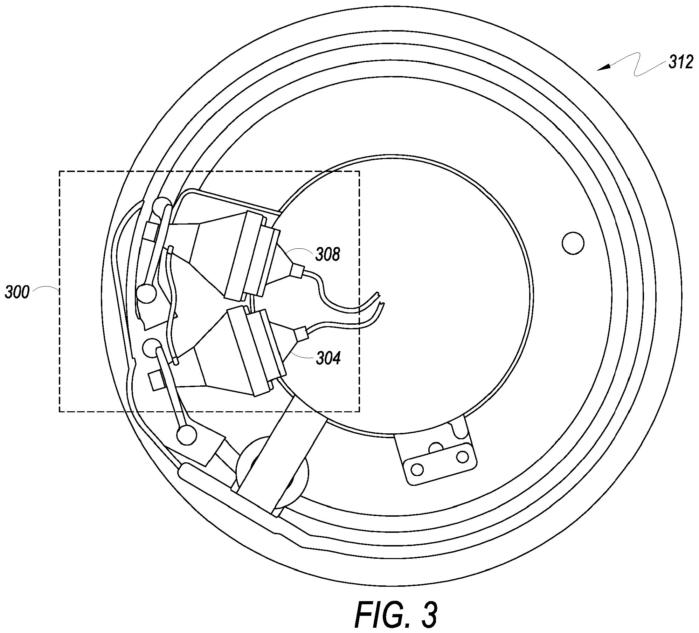

FIG. 3 illustrates an embodiment of a separation chamber system 300 utilizing two chambers 304 and 308. Some embodiments provide for system 300 where two chambers are used to process a stream of cells that includes beads. In these embodiments, a first chamber, such as chamber 304 may be used to initially remove beads from the fluid stream. In embodiments, chamber 308 is utilized primarily to wash and concentrate cells but may also be used to separate out any beads that may remain.

As illustrated in FIG. 3, the separation chamber system 300 may be mounted on a centrifuge rotor 312. The centrifuge rotor may then spin creating a centrifugal field within each of chambers 304 and 308. As a result of the centrifugal field created in each of the chambers, the beads may be separated from the cells in chamber 304 and the cells may be concentrated and washed in chamber 308. It is noted that in some embodiments, only a single separation chamber may be used to separate beads from cells, wash cells, and concentrate cells.

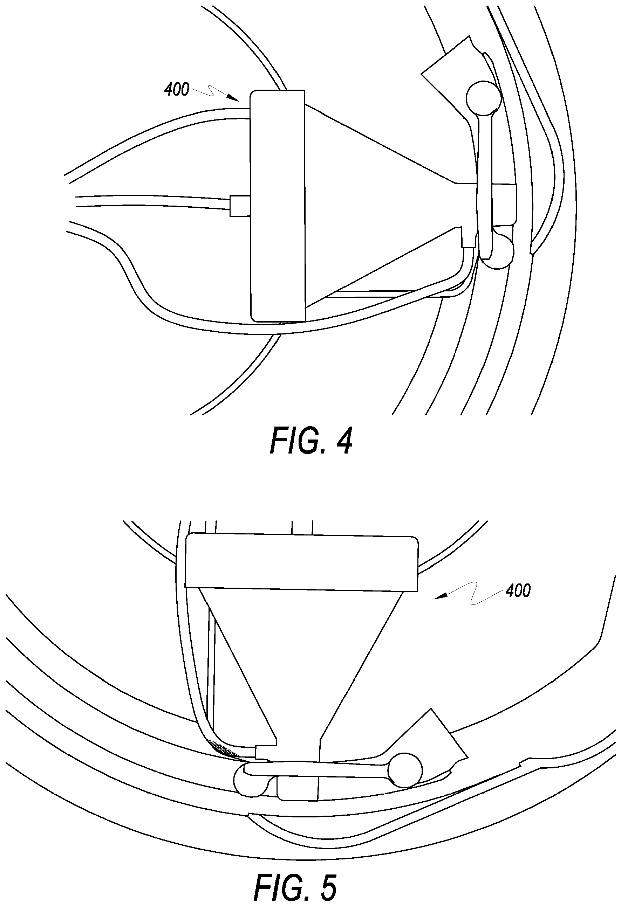

FIGS. 4 and 5 illustrate a separation chamber 400. As illustrated in FIGS. 4 and 5, chamber 400 includes a three port chamber design. In embodiments, the ports of chamber 400 may include a first top port, a second bottom port, and a third side port.

In some embodiments, chamber 400 may be used by transferring a fluid stream containing cells, beads, and liquid into a volume of the chamber. The fluid stream may enter into the chamber through a side port, for example. While in the chamber, the heavier particles, e.g., the beads, may settle to the bottom of the chamber. The chamber 400 may be subjected to a centrifugal field that aids in the beads settling to the bottom of the chamber. The cells may be in an intermediate layer and liquid at the top of the chamber.

A small flow of liquid may enter the chamber through the bottom port to ensure that the cells do not stay attached to beads. The liquid may also be used to wash the cells. FIG. 5 illustrates a close-up view of a bottom portion of chamber 400. As shown in FIG. 5, some beads have settled in the tubing line attached to the bottom port of chamber 400.

Depending on the specific embodiment, once separated, the various components of the fluid stream may be removed from the chamber through any one of the ports. In embodiments, the liquid separated in the chamber may be removed from the chamber 400 through the top port. In some embodiments, the beads may be removed through the bottom port. In other embodiments, the cells may also be removed from the bottom port after the beads have been removed.

The various ports of the chambers may be connected to containers for directing the components of the fluid stream separated in the chamber. For example, the port through which the excess liquid is removed may be connected to a liquid waste bag for storing liquid waste. In other embodiments, the port through which the beads are removed may be directed to a bag to store the beads for disposal or recycling. Finally, the port through which the cells are removed may be connected to a fill system, such as system 116 (FIG. 1) which may direct a stream of concentrated cells to individual containers. A storage solution such as DMSO may also be added to the individual containers.

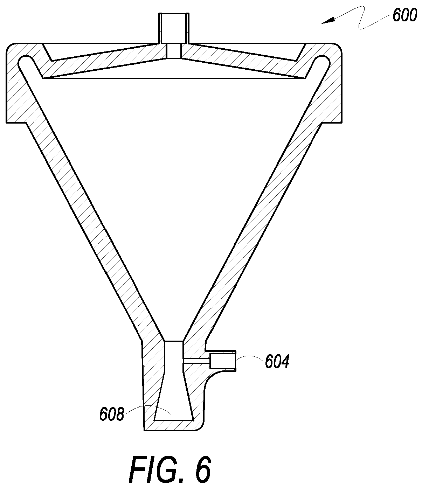

FIG. 6 illustrates a cross-sectional view of another separation chamber 600 that may be used to separate beads from cells. As illustrated in FIG. 6, chamber 600, includes trap 608 and a port 604, which may in embodiments be an inlet, an outlet, or an inlet/outlet port. In embodiments, chamber 600 is positioned on a rotor of a centrifuge which creates a centrifugal field. When a stream of fluid is transferred into chamber 600, the heaviest particles in the fluid, e.g., beads, will settle into trap 608 where they will remain. Any of the other components (e.g., liquid, cells, etc.) can then be removed from chamber 600 through port 604.

Chambers 400 and 600 are provided for illustrative purposes only. It is noted that other chamber designs may be used in other embodiments. Some embodiments, may utilize one or more of the chamber designs disclosed and described in U.S. Patent Publication No. US 2006/0045921 A1 noted above.

As previously noted, embodiments provide for a flow cell to be used to remove beads. In one specific embodiment, the beads may be magnetic beads that may contain a magnetic material, e.g., iron, nickel, cobalt, some rare earth metals or alloys thereof. In one specific embodiment, the beads may be made from a magnetic material coated with a polymer, such as for example DYNABEADS. In these embodiments, the flow cell may include magnets, e.g., an array of magnets that may be used to remove the magnetic beads from a fluid stream. Details of some embodiments of flow cells are described below.

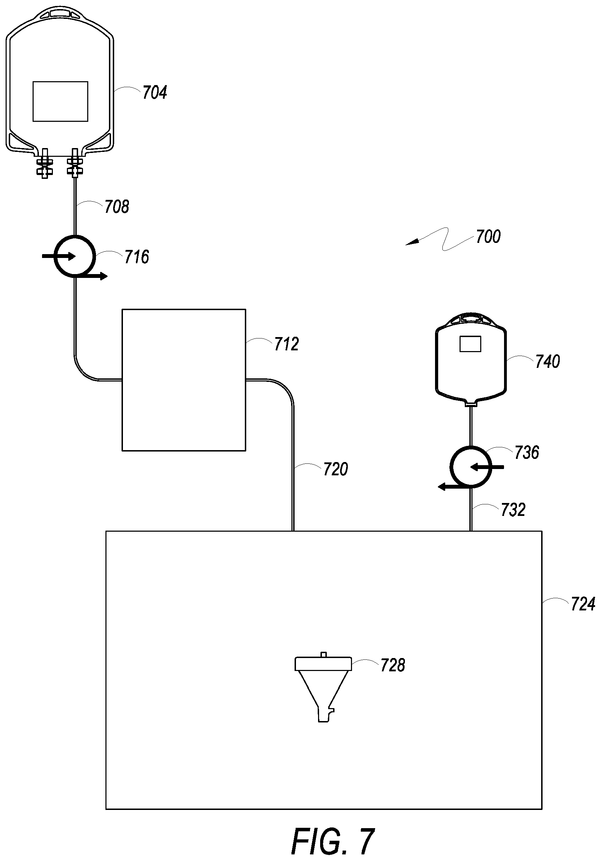

FIG. 7 illustrates a block diagram of one embodiment of a separation/concentration system 700 that utilizes a flow cell 712 for separating beads from a fluid stream that includes beads and cells. System 700 includes a source of fluid 704 and tubing 708 that connects the source of fluid to a flow cell 712. Pump 716, pumps the fluid stream from source 704 into a fluid pathway that includes flow cell 712, where beads may be removed from the fluid stream. As described in greater detail below, the flow cell 712, in embodiments, may utilize magnets, e.g., magnet arrays, to remove magnetic beads from the fluid stream.

After beads have been removed from the fluid stream, cells in the fluid stream may be concentrated and/or washed. For example, the de-beaded fluid stream may be moved into concentrator 724 through tubing 720, where a chamber 728 may be used to concentrate the cells. The chamber may be for example one of chambers 400 or 600 described above. In other embodiments, it may be a three-port chamber such as one or more of the chambers described in U.S. Patent Publication No. US 2006/0045921 A1 noted above. In addition to concentrating the cells, a wash fluid may be added to the cells while in the chamber 728 to wash the cells. The chamber 728 may also remove any beads remaining in the fluid.

After the cells have been concentrated/washed, the stream of cells may be moved through tubing 732 by pump 736 into storage bag 740 for storage. In other embodiments, tubing 732 may be connected to a fill system, such as system 116 that may direct the stream of cells into more than one storage bag for storage until use.

It is noted that system 700 is being provided merely for illustrative purposes. As noted, in some embodiments, system 700 may be incorporated into a larger system such as system 100. In other embodiments, system 700 may operate as a stand-alone system. Also, the system may include additional components in some embodiments. For example, below are some examples of specific features of a flow cell that may be used in some embodiments. In other embodiments, system 700 may include fewer than the components shown in FIG. 7. For example, in some embodiments, system 700 may not include concentrator 724 and may be used merely to remove beads from a fluid that may include the beads and cells. In other embodiments, portions of system 700 may be integrated into assemblies such as assembly 200 (FIG. 2).

In some embodiments, system 700 may be used to process a variety of volumes of fluid. As one example, fluid source 704 may store from about 10 ml to about 5000 ml, which is processed by system 700. In one embodiment, system 700 may process volumes from about 1 ml to about 5000 ml, such as about 10 ml to about 2500 ml, such as about 100 ml to about 2000 ml, or even about 150 ml to about 1500 ml. In embodiments, system 700 may process volumes greater than about 10 ml, greater than about 20 ml, greater than about 30 ml, greater than about 40 ml, greater than about 50 ml, greater than about 60 ml, greater than about 70 ml, greater than about 80 ml, greater than about 90 ml, or even greater than about 100 ml. In embodiments, system 700 may process volumes less than about 5000 ml, less than about 4000 ml, less than about 3000 ml, less than about 2000 ml, or even less than about 1000 ml.

Flow rates though a system such as 700 and its components, e.g., through flow cell 712, may be from about 1 ml/min to about 500 ml/min, such as about 5 ml to about 250 ml, such as about 10 ml/min to about 200 ml, or even about 15 ml to about 150 ml. In embodiments, system 700 may process volumes at rates greater than about 10 ml/min, greater than about 20 ml/min, greater than about 30 ml/min, greater than about 40 ml/min, or even greater than about 50 ml/min. In embodiments, flow rates may be less than about 500 ml/min, less than about 400 ml/min, less than about 300 ml/min, less than about 200 ml/min, or even less than about 100 ml/min.

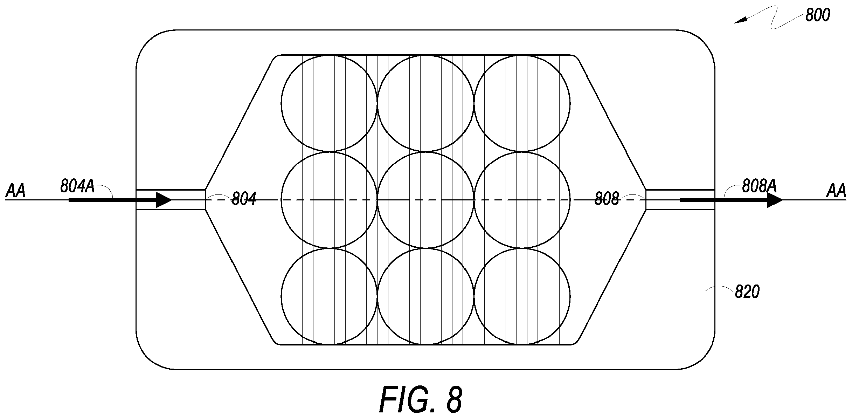

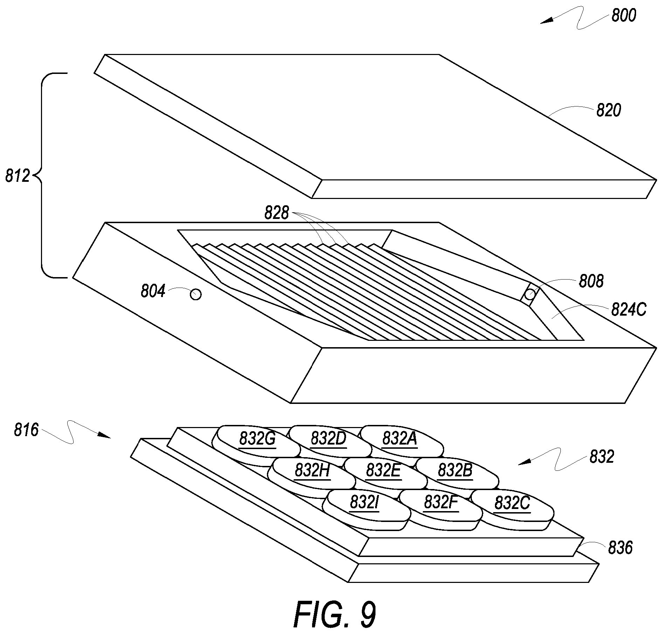

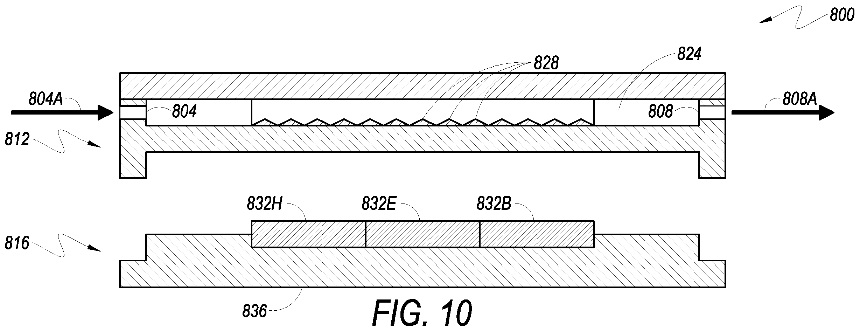

FIGS. 8-10 illustrate various views of a flow cell 800 that may be used in some embodiments not covered by the claimed invention to remove beads, e.g., magnetic beads from a fluid stream that includes the beads and cells. Flow cell 800 may be used in systems such as system 700 (FIG. 7) as flow cell 712. FIG. 8 illustrates a top view, FIG. 9 illustrates an exploded view and FIG. 10 illustrates a cross-sectional (taken through line AA) and partially exploded view (FIG. 8).

As shown in FIG. 8, liquid flows into flow cell 800 through inlet port 804, in the direction illustrated by arrow 804A, and flows out of cell 800 through exit port 808 in the direction illustrated by arrow 808A. As described in greater detail below, the features of flow cell 800 remove beads from the fluid stream, which results in a de-beaded fluid stream exiting flow cell 800 through exit port 808.

As illustrated in FIGS. 8-10, flow cell 800 includes a top portion 812 and a bottom portion 816 (FIG. 9). The top portion 812 includes top wall 820 that helps to define a chamber 824 through which a fluid stream flows. Within the chamber 824 there are a plurality of ridges 828 that are designed to create turbulent flow as the fluid stream passes over them.

Bottom portion 816 includes an array 832 of magnets (832A-I) that is positioned adjacent, e.g., underneath, the chamber 824 and ridges 828. The magnets 832A-I are held in place by piece 836 and are configured to attract magnetic beads in the fluid stream and maintain the beads against a wall of the chamber 824, e.g., a bottom wall.

The ridges 828 may aid in removing beads from the fluid stream. Without being bound by theory, it is believed that the ridges 828 may create eddy currents in the stream as it passing over the ridges 828. As a result, the heavier particles, namely the beads will have a tendency to settle at the bottom of the ridges. The eddy currents may also separate cells that may be stuck to beads. Furthermore, the magnetic array 832 may attract the beads and trap them at the bottom of the ridges, removing them from the stream as the stream flows through the flow cell 800.

Flow cell 800 may be utilized prior to flowing a stream of cells into a chamber, such as a chamber for cell concentration and/or wash (e.g., chamber 400, chamber 600, and/or chamber 728). A stream of fluid would flow through a flow cell 800 to remove a majority of the beads in the stream, after which the flow would be directed to a chamber where the cells could be washed and concentrated and any remaining beads removed such as is described above with respect to FIG. 7.

There may be additional wash solution that is flowed into and out of flow cell 800. For example, a wash solution could be flowed backward through flow cell 800 (after an initial stream has been treated in flow cell 800). That is, wash solution could flow into flow cell 800 in the opposite direction of arrow 808A and flowed out of the flow cell 800 in the opposite direction of arrow 804A. The wash solution may remove any cells that are in flow cell 800 and direct the flow to a chamber for further washing and concentrating of any cells picked up by the wash solution.

Alternatively, after the wash solution is flowed in the opposite direction, it could be reflowed in the directions illustrated by arrows 804A and 808A. This would allow the wash solution to flow through the flow cell 800 twice to remove cells that remained in flow cell 800 after the initial treatment of a stream of fluid. The wash solution may flow only once through flow cell 800 and it may be in an opposite direction than illustrated by arrows 804A and 808A, or in the same direction.

The top portion and bottom portion of flow cell 800 may be made from any suitable material some non-limiting examples including polyvinyl chloride, polyethylene, polyethylene terephthalate, polypropylene, and combinations thereof. The material for flow cell 800 may be transparent and/or translucent to allow a user to visually determine whether beads are being removed from a fluid stream.

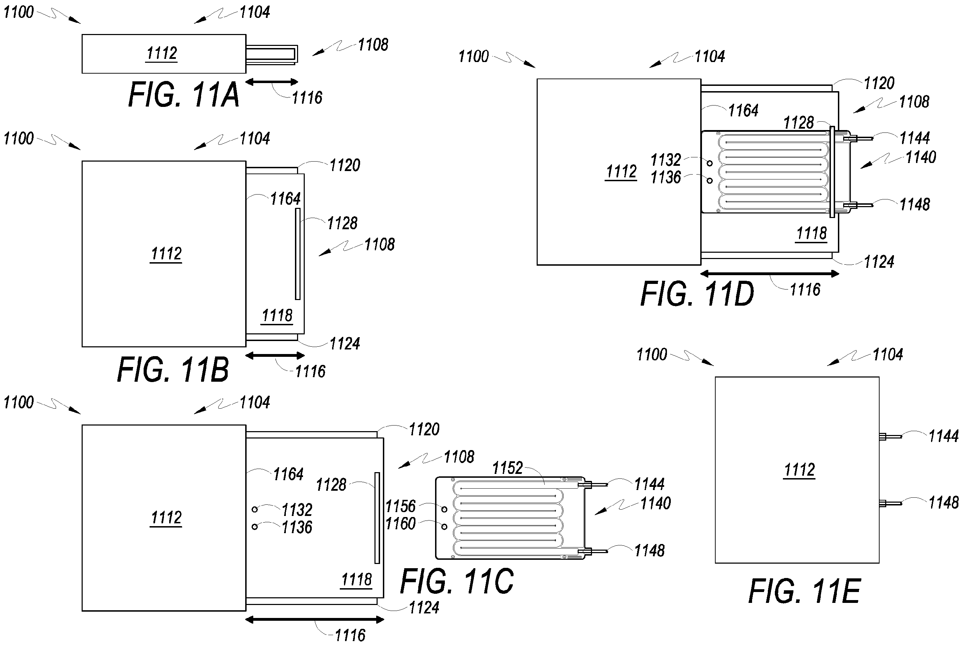

FIGS. 11A-E illustrate various views of a flow cell 1100 that are used in some embodiments of the invention to remove beads, e.g., magnetic beads from a fluid stream that includes the beads and cells. In some embodiments, flow cell 1100 may be used in systems such as system 700 (FIG. 7) as flow cell 712. FIG. 11A illustrates a top view of flow cell 1100 showing a stationary portion 1104 and a slideable portion 1108 positioned inside a housing 1112 of the stationary portion 1104. As can be appreciated, in embodiments, slideable portion 1108 may be moved in and out of housing 1112 as illustrated by arrow 1116.

FIGS. 11B-E illustrate side views of flow cell 1100. As illustrated in FIGS. 11B-E, slideable portion 1108 includes plate 1118, rails 1120 and 1124 that allow plate 1118 to slide in and out of housing 1112. As may be appreciated, rails 1120 and 1124 engage tracks inside housing 1112 that guide the rails 1120 and 1124 during sliding.

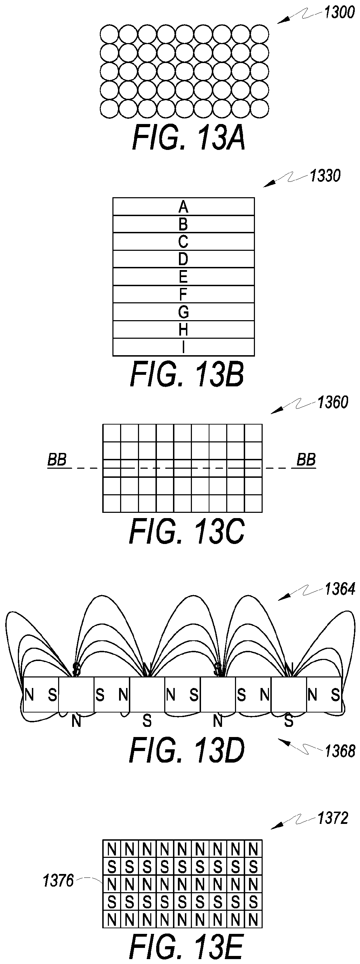

Slideable portion 1108 includes a plate 1118. In embodiments of the invention, plate 1118 includes a magnet, or an array of magnets that aid in removing magnetic beads from a fluid stream passed through flow cell 1100. In other embodiments not covered by the invention, the magnets may be on an interior wall of housing 1112, such as inside surface 1164 of housing 1112. Examples of some magnets and/or magnetic arrays that may be included as part of plate 1118 and/or interior wall 1164 are described below with respect to FIGS. 13A-E.

In embodiments, plate 1118 may be made of a magnetic material and magnetized (e.g., with areas of different polarity to create arrays, for example as shown in FIGS. 13E and 13F). In other embodiments, plate 1118 may be constructed of multiple materials/pieces such as a base plate that then may secure a magnetic material, such as a magnet or an array of magnets with fasteners. The base plate may be made of a polymeric (e.g., polyethylene), metallic (e.g., aluminum), or a combination of materials.

As illustrated in FIGS. 11C-E, embodiments provide for the use of a bag (e.g., 1140) with an indirect flow path. By a bag with an indirect flow path, it is meant that a flow path between an inlet (e.g., 1144) and outlet (e.g., 1148) of the bag is not in a substantially straight line (e.g., tortuous path, path with barriers, path with baffles, etc.). In FIGS. 11C-E the bag is a serpentine bag 1140 with a tortuous path between the inlet and outlet that may be created by the walls of a channel.

In embodiments, the serpentine bag 1140 may be a disposable portion that may be attached to the movable portion 1108 as described in greater detail below. As illustrated in FIG. 11C serpentine bag 1140 includes an inlet port 1144 and an outlet port 1148. Bag 1140 also include channel 1152 through which a fluid stream is moved, such as for example by a pump orgravity. The channel 1152 provides part of a fluid pathway for the fluid stream with the channel walls providing barriers that create an indirect path. In addition, serpentine bag 1140 may have mechanisms for attaching bag 1140 to portion 1108. For example, perforations 1156 and 1160 may be used to secure serpentine bag to portion 1108. Plate 1118 may include corresponding mechanisms such as post 1132 and post 1136 that may fit in the perforations 1156 and 1160. In addition, plate 1118 may include a clamp 1128 to further secure the serpentine bag 1140 on plate 1118.

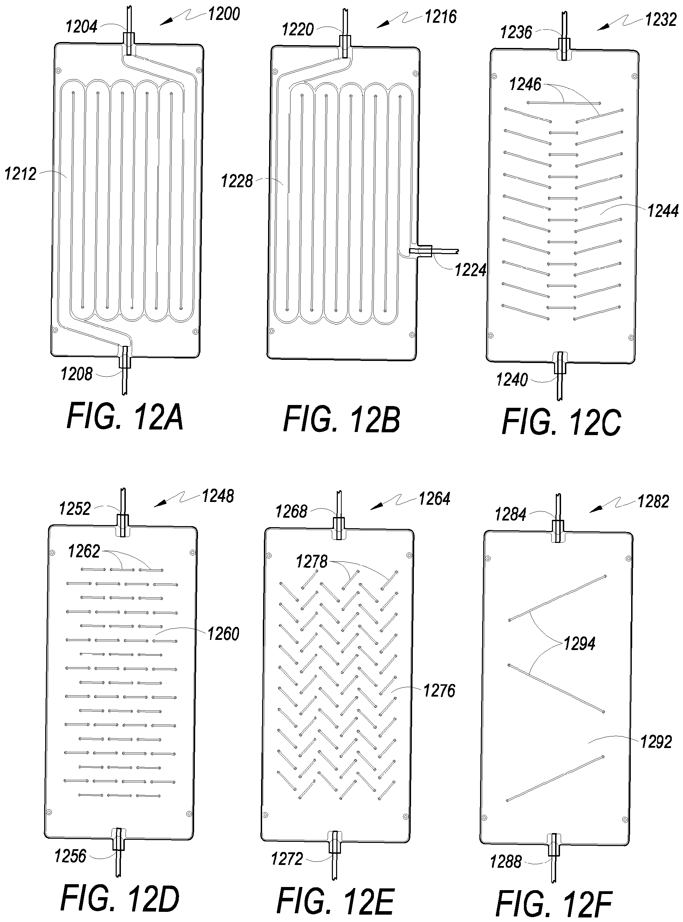

Although serpentine bag 1140 is shown as having a particular design, embodiments are not necessarily limited thereto. FIGS. 12A and 12B illustrate alternative serpentine bag designs that may be used with some embodiments. Additionally, FIGS. 12C-F illustrate other examples of bags with indirect flow paths that may be used in embodiments.

Referring to FIGS. 12A and 12B, each of bags 1200 (FIG. 12A) and 1216 (FIG. 12B) may be used in some embodiments as part of a flow cell. As shown in FIG. 12A bag 1200 includes an inlet port 1204 and an outlet port 1208. Bag 1200 also includes channel 1212 through which a fluid stream is moved, such as for example by a pump or gravity. Similarly, FIG. 12B illustrates bag 1216, which includes an inlet port 1220 and an outlet port 1224. Bag 1216 includes channel 1228 through which a fluid stream is moved, such as for example by a pump or gravity.

Although similar, bags 1200 and 1216 do have some different features. For example, the inlet ports and outlet ports are located at different positions. Bag 1200 has the inlet port 1204 located on a side parallel to the side on which the outlet port 1208 is located. In contrast, bag 1216 illustrates inlet port 1220 on a side perpendicular to the side on which the outlet port 1224 is located. Furthermore, channel 1212 is slightly longer than the channel 1228. These are merely some examples, and embodiments may provide for different channel lengths, diameters, different port locations, etc.

Referring to FIG. 12C, bag 1232 includes inlet port 1236 and outlet port 1240. In addition, indirect flow path 1244 is created, in part, by barriers 1246. A fluid stream that enters inlet port 1236 must flow around barriers 1246 to reach outlet port 1240. Accordingly, the flow path 1244 is indirect, which maintains the fluid in bag 1232 for a longer period of time than a direct path would. The additional time allows a magnetic field (e.g., generated by magnets on plate 1118) to act on the fluid stream for a longer period of time to remove magnetic beads from the fluid stream.

FIG. 12D illustrates another bag 1248 that includes inlet port 1252 and outlet port 1256. An indirect flow path 1260 is created, in part, by barriers 1262. A fluid stream that enters inlet port 1252 must flow around barriers 1262 to reach outlet port 1256. Accordingly, the flow path 1260 is indirect, which maintains the fluid in bag 1248 for a longer period of time than a direct path would.

FIG. 12E illustrates another bag 1264 that includes inlet port 1268 and outlet port 1272. An indirect flow path 1276 is created, in part, by barriers 1278. A fluid stream that enters inlet port 1268 must flow around barriers 1278 to reach outlet port 1272. Accordingly, the flow path 1276 is indirect and maintains the fluid in bag 1264 for a longer period of time than a direct path would allowing more time for a magnetic field (e.g., generated by magnets on plate 1118) to act on the fluid stream.

FIG. 12F illustrates yet another bag 1282 that includes inlet port 1284 and outlet port 1288. An indirect flow path 1292 is created, in part, by barriers 1294. A fluid stream that enters inlet port 1284 must flow around barriers 1294 to reach outlet port 1288. Accordingly, the flow path 1292 is indirect and maintains the fluid in bag 1282 for a longer period of time than a direct path would allowing more time for a magnetic field (e.g., generated by magnets on plate 1118) to act on the fluid stream.

In embodiments, the indirect fluid flow path of a bag may be provided by any feature that makes the fluid flow path long enough to allow a magnetic field to remove magnetic beads from the fluid stream while flowing in the bag. In embodiments, the indirect fluid flow path should be long enough to allow removal of greater than about 80%, greater than about 90%, greater than about 95%, greater than about 98% or even greater than about 99% of beads from a volume of fluid flowed through the bag. In other embodiments, the flow path may be long enough to remove less than about 99%, less than about 98% or even less than about 97% of the beads in a volume of fluid flowed through the bag.

In embodiments, bags (1140, 1200, 1216, 1232, 1248, 1264, and 1282) may be made of two sheets of polymeric material that are sealed together such as by radio frequency welding, acoustic welding, laser welding, and/or solvent welding, etc. In some embodiments, the features of the flow paths, e.g., channels 1152, 1212, and/or 1228 and barriers 1246, 1262, 1278, and 1294) may be formed by sealing the two sheets in a pattern to form the features. The sheets may be made from any suitable material some non-limiting examples including polyvinyl chloride, polyethylene, polyethylene terephthalate, polypropylene, and combinations thereof.

In embodiments, bags 1140, 1200, 1216, 1232, 1248, 1264, and 1282 include additional features that may aid in the removal of beads from a fluid stream. The bags 1140, 1200, 1216, 1232, 1248, 1264, and 1282 are made of sheets of polymeric material which include a texture. As one example, the texture may include ridges similar to ridges 828 (FIG. 8). In other examples, the texture may be a regular or random pattern of features that provide some roughness to the surface over which a fluid stream flows. The texture may in embodiments assist in maintaining beads against a wall of the bag (e.g., 1140, 1200, 1216, 1232, 1248, 1264, and 1282). The texture may in embodiments affect the flow of fluid in the bag, such as by creating eddy currents in the stream as it passing over the texture moving liquid toward the wall where the magnetic field is located. As another example, the bag may be transparent and/or translucent to allow a user to visually determine whether beads are being maintained on a wall of the bag.

Referring back to FIGS. 11C-E, serpentine bag 1140 may be mounted onto portion 1108 by first sliding portion 1108 out of housing 1112 (FIG. 11C). Serpentine bag 1140 may then be positioned onto portion 1108 by inserting posts 1132 and 1136 into perforations 1156 and 1160 respectively (FIG. 11D). Additionally, clamp 1128 may further secure the serpentine bag onto plate 1118. Finally, the slideable portion 1108 may be moved back into housing 1112 (FIG. 11E).

Once bag 1140 is attached to plate 1118 and positioned in housing 1112, bag 1140 may be subjected to a magnetic field. The magnetic field may be generated by magnets or an array of magnets on plate 1118 or on the inside surface 1164 of housing 1112. Embodiments provide for the magnets or arrays to be arranged to maximize the magnetic field to attract the beads in the fluid stream when it passes through the channel.

The inlet port 1144 and outlet port 1148 of bag 1140 may be fluidly connected to other components of a system to process a stream of liquid. For example, as described above with respect to FIG. 7, inlet port 1144 may be attached to liquid source 704 and outlet port 1148 may be attached to concentrator 724.

Examples of magnetic arrays that may be used in some embodiments are illustrated in FIGS. 13A-E. FIG. 13A illustrates an array 1300 of flat round magnets, similar to array 832 of flow cell 800 (FIG. 8-10). Array 1300 includes forty-five magnets that are arranged in five rows. In other embodiments, array 1300 may have a different number of magnets that may be arranged in different patterns.

FIG. 13A illustrates array 1330 which includes rectangular plate magnets 1330A-I, which are arranged in a single column. In other embodiments, the magnets may be arranged in different patterns. For example, one or more of magnets 1330A-I may be positioned perpendicular to one or more other magnets 1330A-I.

FIG. 13C illustrates an array 1360 square magnets. Array 1360 includes forty-five magnets that are arranged in five rows. In other embodiments, array 1360 may have a different number of magnets that may be arranged in different patterns.

In embodiments, arrays may be arranged to control the characteristics of the magnetic field. For example, an array may be arranged in a Halbach array to create a strong magnetic field adjacent the serpentine bag. FIG. 13D illustrates a cross-sectional view of array 1360 (taken through line BB), which is arranged in a Halbach array. As illustrated in FIG. 13D, the arrangement results in augmentation of the magnetic field on a top surface of array 1360 creating strong magnetic field 1364 and in cancelation of the magnetic field on a bottom surface creating weak magnetic field 1368. Embodiments may provide for a flow cell to be configured so that a serpentine bag is positioned adjacent a magnetic field similar to strong magnetic field 1364 to maintain magnetic beads on a wall of the serpentine bag as a fluid stream with magnetic beads and cells flows through the bag.

FIG. 13E illustrates another array 1372 that includes square magnets. The magnets in array 1372 are arranged with alternating poles. FIG. 13E illustrates a top view showing the poles on a top surface 1376. As may be appreciated, a bottom surface would have a corresponding opposite pole. In embodiments, array 1372 may provide a magnetic field that enhances removal of magnetic beads from a fluid stream.

The arrangements of magnets described above are provided merely for illustrative purposes. Other embodiments may provide for different shapes, sizes, and arrangements of magnets that may affect the characteristics of the magnetic field used in a flow cell. Also, in other embodiments, the magnets may be permanent magnets or electro magnets. In addition to the magnets themselves, arrays may additionally include the use of other materials such as ferromagnetic materials to separate magnets, which may help in controlling the shape, strength, or size of the magnetic field used to trap the beads.

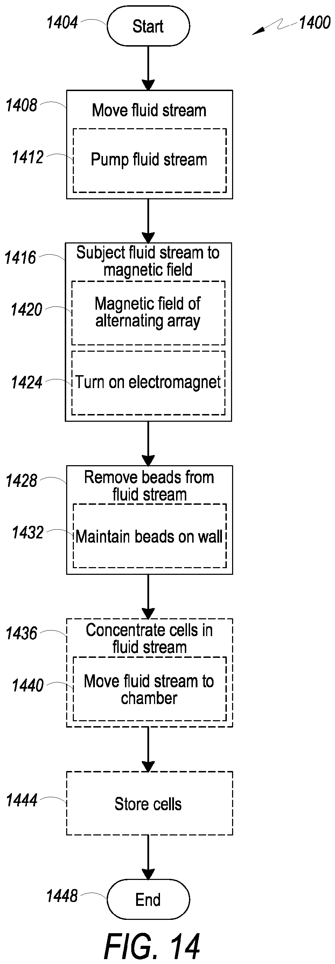

FIG. 14 illustrates a flow chart 1400 for a process that may be performed in embodiments not covered by the present invention. Although specific components may be described below for performing steps in flow chart 1400, the process is not limited thereto. For example, some steps may be described as performed by a flow cell (e.g., 712, 800, 1100), while others are described as performed by a pump (e.g., 716). This is done merely for illustrative purposes, and flow chart 1400 is not limited to being performed by any specific device or component(s).

Flow chart 1400 may be performed to process a fluid stream, such as a stream containing cells (e.g., T cells) and magnetic beads. The process may involve removing magnetic beads from the fluid stream that includes the cells. Flow 1400 starts at 1404 and passes to step 1408 where a fluid stream is moved through a fluid pathway. The fluid pathway includes an indirect pathway in a bag. Step 1408 may involve some sub-steps. For example, the fluid may involve optional sub-step 1414 where the fluid is pumped, e.g., using pump 716. Alternatively, the fluid may be moved at step 1408 by gravity.

Step 1408 may involve moving the fluid stream of beads and cells at a predetermined rate. For example, the fluid may be moved at rates from about 1 ml/min to about 500 ml/min, such as about 5 ml to about 250 ml, such as about 10 ml/min to about 200 ml, or even about 15 ml to about 150 ml. Rates may be greater than about 10 ml/min, greater than about 20 ml/min, greater than about 30 ml/min, greater than about 40 ml/min, or even greater than about 50 ml/min. Flow rates may be less than about 500 ml/min, less than about 400 ml/min, less than about 300 ml/min, less than about 200 ml/min, or even less than about 100 ml/min.

After step 1408, flow 1400 passes to step 1416, where the fluid stream is subjected to a magnetic field. Step 1416 may involve the use of a flow cell such as flow cells, 712, 800, and/or 1100 that include magnets or arrays of magnets. Step 1416 may involve some sub-steps. Step 1416 may involve subjecting the fluid stream to a magnetic field while flowing through a bag.

Step 1416 may involve some sub-steps. For example, at sub-step 1420, the fluid stream may be subjected to a magnetic field generated by an alternating array. The array may be similar to array 1372 (FIG. 13E) or have a different arrangement of magnets. Alternatively, the magnetic field used in step 1416 may be generated by an electromagnet. Herein sub-step 1424 may be performed to turn the electromagnet on to create the magnetic field.

After step 1416, beads are removed from the fluid stream at step 1428. As noted above, the beads may be magnetic and as a result of being subjected to the magnetic field at step 1416, the beads may be removed from the fluid stream. Step 1428 may involve some sub-steps. For example, sub-step 1432 may involve having the magnetic field attract the beads and maintain them against the wall of a bag while the fluid stream flows through the bag.

During step 1428, a large portion of beads in a volume of fluid may be removed without losing a large portion of the cells. Greater than about 75%, greater than about 80%, greater than about 85%, greater than about 90%, greater than about 95%, greater than about 96%, or even greater than about 98% of beads may be removed from a volume of fluid. Alternatively, step 1428 may remove less than about 99%, less than about 98% or even less than about 97% of the beads in a volume of fluid flowing through the bag.

After step 1428, optional step 1436 may be performed to concentrate cells in the fluid stream. Step 1436 may be performed by a concentrator/washing system (e.g., 108 and 724). Step 1436 may involve some sub-steps, such as sub-step 1440 where fluid is moved into a chamber where the cells are concentrated. The chamber may be a three port chamber where cells may be concentrated and washed (e.g., 400 and 600). Additionally, some remaining beads may be removed from the fluid stream by the chamber.

After optional step 1436, the cells may be stored at optional step 1444. After removing beads from a volume of fluid, greater than about 90%, greater than about 95%, greater than about 96%, greater than about 97%, greater than about 98% or even greater than about 99% of the cells may be recovered from a volume of solution and be used, e.g., for therapeutic purposes. Alternatively, less than about 99%, less than about 98%, less than about 97%, less than about 96% or less than about 95% of the cells in a volume of fluid may be recovered. Flow 1400 ends at 1448.

Although flow 1400 has been described with steps listed in a particular order, the process is not limited thereto. Steps may be performed in different order, in parallel, or any different number of times, e.g., before and after another step. Also, as indicated above, flow 1400 includes some optional steps. However, those steps above that are not indicated as optional should not be considered as essential to the Process.

It will be apparent to those skilled in the art that various modifications and variations can be made to the methods and structure of the present invention without departing from its scope which is defined by the appended claims. Thus, it should be understood that the invention is not limited to the specific examples given. Rather, the invention is intended to cover modifications and variations within the scope of the following claims.

While example embodiments and applications of the present invention have been illustrated and described, it is to be understood that the invention is not limited to the precise configuration and resources described above. Various modifications, changes, and variations apparent to those skilled in the art may be made in the arrangement, operation, and details of the methods and systems of the present invention disclosed herein without departing from the scope of the claimed invention.

## Claims

1. A system for removing magnetic beads from a fluid stream comprising cells, the system comprising:
a bag (1140, 1200, 1216, 1232, 1248, 1264, 1282) comprising a fluid pathway for flowing the fluid stream, wherein the fluid pathway comprises an indirect pathway (1212, 1228, 1244, 1260) in the bag (1140, 1200, 1216, 1232, 1248, 1264, 1282), the indirect pathway (1212, 1228, 1244, 1260) not being in a substantially straight line between an inlet (1204, 1220, 1236, 1252, 1268, 1284) and an outlet (1208, 1224, 1240, 1256, 1272, 1288) of the bag (1140, 1200, 1216, 1232, 1248, 1264, 1282), wherein the bag (1140, 1200, 1216, 1232, 1248, 1264, 1282) is made of sheets of polymeric material that include a texture;
a flow cell (1100) comprising:
a stationary portion (1104) with a housing (1112); and
a slidable portion (1108) with a plate (1118) and rails (1120) that engage tracks inside the housing (1112) to allow the plate (1118) to slide in and out of the housing (1112), wherein the plate (1118) includes a magnet that creates a magnetic field (1364) adjacent the bag (1140, 1200, 1216, 1232, 1248, 1264, 1282); and a pump (716, 736) for moving fluid through the fluid pathway.

2. The system of claim 1, wherein the plate (1118) comprises an array of magnets (832).

3. The system of claim 1, wherein the indirect pathway comprises a tortuous path (1212, 1228, 1244, 1260) between the inlet (1204, 1220, 1236, 1252, 1268, 1284) and the outlet (1208, 1224, 1240, 1256, 1272, 1288).

4. The system of claim 1, wherein the bag (1140, 1200, 1216, 1232, 1248, 1264, 1282) comprises barriers (1246, 1262, 1278, 1294) around which the fluid stream flows.

5. The system of claim 2, wherein the array of magnets is arranged in a Halbach array (1360).

6. The system of claim 1, wherein the texture assists in maintaining the magnetic beads against a wall of the bag (1140, 1200, 1216, 1232, 1248, 1264, 1282).

7. The system of claim 1, wherein the pump (716, 736) is configured to move fluid into the bag (1140, 1200, 1216, 1232, 1248, 1264, 1282) at a rate greater than 10 ml/min.

8. The system of claim 1, further comprising a chamber (728) for concentrating the cells in the fluid stream, wherein the outlet of the bag is in fluid communication with the chamber (728) for concentrating the cells in the fluid stream after the magnetic beads are removed from the fluid stream.

9. The system of claim 8, wherein the chamber (728) comprises a top port, a bottom port, and a side port.

10. The system of claim 9, wherein the fluid stream enters the chamber through the side port.

11. The system of claim 1, wherein the plate (1118) comprises posts (1132, 1136) for attaching the bag (1140, 1200, 1216, 1232, 1248, 1264, 1282).

12. The system of claim 1, wherein the plate (1118) comprises a clamp (1128).

## Patentansprüche

1. System zum Entfernen von magnetischen Kügelchen aus einem Fluidstrom, der Zellen umfasst, wobei das System Folgendes umfasst:
einen Beutel (1140, 1200, 1216, 1232, 1248, 1264, 1282), umfassend einen Fluidweg zum Fließen des Fluidstroms, wobei der Fluidweg einen indirekten Weg (1212, 1228, 1244, 1260) in dem Beutel (1140, 1200, 1216, 1232, 1248, 1264, 1282) umfasst, wobei der indirekte Weg (1212, 1228, 1244, 1260) nicht in einer im Wesentlichen geraden Linie zwischen einem Einlass (1204, 1220, 1236, 1252, 1268, 1284) und einem Auslass (1208, 1224, 1240, 1256, 1272, 1288) des Beutels (1140, 1200, 1216, 1232, 1248, 1264, 1282) verläuft, wobei der Beutel (1140, 1200, 1216, 1232, 1248, 1264, 1282) aus Folien aus Polymermaterial hergestellt ist, die eine Struktur beinhalten;
eine Durchflusszelle (1100), umfassend:
einen stationären Abschnitt (1104) mit einem Gehäuse (1112); und
einen verschiebbaren Abschnitt (1108) mit einer Platte (1118) und Schienen (1120), die mit Spuren in dem Gehäuse (1112) in Eingriff sind, um zuzulassen, dass sich die Platte (1118) in das Gehäuse (1112) und daraus heraus verschiebt, wobei die Platte (1118) einen Magneten beinhaltet, der ein Magnetfeld (1364) neben dem Beutel (1140, 1200, 1216, 1232, 1248, 1264, 1282) erzeugt; und eine Pumpe (716, 736) zum Bewegen von Fluid durch den Fluidweg.

2. System nach Anspruch 1, wobei die Platte (1118) eine Anordnung von Magneten (832) umfasst.

3. System nach Anspruch 1, wobei der indirekte Weg einen gewundenen Weg (1212, 1228, 1244, 1260) zwischen dem Einlass (1204, 1220, 1236, 1252, 1268, 1284) und dem Auslass (1208, 1224, 1240, 1256, 1272, 1288) umfasst.

4. System nach Anspruch 1, wobei der Beutel (1140, 1200, 1216, 1232, 1248, 1264, 1282) Barrieren (1246, 1262, 1278, 1294) umfasst, um die der Fluidstrom fließt.

5. System nach Anspruch 2, wobei die Anordnung von Magneten in einem Halbach-Array (1360) angeordnet ist.

6. System nach Anspruch 1, wobei die Struktur dabei hilft, die magnetischen Kügelchen gegen eine Wand des Beutels (1140, 1200, 1216, 1232, 1248, 1264, 1282) zu halten.

7. System nach Anspruch 1, wobei die Pumpe (716, 736) konfiguriert ist, um Fluid in den Beutel (1140, 1200, 1216, 1232, 1248, 1264, 1282) bei einer Geschwindigkeit von mehr als 10 ml/min zu bewegen.

8. System nach Anspruch 1, ferner umfassend eine Kammer (728) zum Konzentrieren der Zellen im Fluidstorm, wobei der Auslass des Beutels in Fluidkommunikation mit der Kammer (728) zum Konzentrieren der Zelle im Fluidstrom ist, nachdem die magnetischen Kügelchen aus dem Fluidstrom entfernt sind.

9. System nach Anspruch 8, wobei die Kammer (728) eine obere Öffnung, eine untere Öffnung und eine seitliche Öffnung umfasst.

10. System nach Anspruch 9, wobei der Fluidstrom die Kammer durch die seitliche Öffnung betritt.

11. System nach Anspruch 1, wobei die Platte (1118) Pfosten (1132, 1136) zum Befestigen des Beutels (1140, 1200, 1216, 1232, 1248, 1264, 1282) umfasst.

12. System nach Anspruch 1, wobei die Platte (1118) eine Klemme (1128) umfasst.

## Revendications

1. Système pour enlever des billes magnétiques à partir d'un courant de fluide comprenant des cellules, le système comprenant :
un sac (1140, 1200, 1216, 1232, 1248, 1264, 1282) comprenant une voie de passage de fluide pour faire écouler le courant de fluide, dans lequel la voie de passage de fluide comprend une voie de passage indirecte (1212, 1228, 1244, 1260) dans le sac (1140, 1200, 1216, 1232, 1248, 1264, 1282), la voie de passage indirecte (1212, 1228, 1244, 1260) n'étant pas dans une ligne sensiblement droite entre une entrée (1204, 1220, 1236, 1252, 1268, 1284) et une sortie (1208, 1224, 1240, 1256, 1272, 1288) du sac (1140, 1200, 1216, 1232, 1248, 1264, 1282), dans lequel le sac (1140, 1200, 1216, 1232, 1248, 1264, 1282) est fait de feuilles de matériau polymère qui incluent une texture ;
une cellule d'écoulement (1100) comprenant :
une partie stationnaire (1104) avec un logement (1112) ; et
une partie coulissante (1108) avec une plaque (1118) et des rails (1120) qui entrent en prise avec des pistes à l'intérieur du logement (1112) pour permettre à la plaque (1118) de coulisser dans le logement (1112) et hors de ce dernier, dans lequel la plaque (1118) inclut un aimant qui crée un champ magnétique (1364) adjacent au sac (1140, 1200, 1216, 1232, 1248, 1264, 1282) ; et une pompe (716, 736) pour déplacer un fluide à travers la voie de passage de fluide.

2. Système selon la revendication 1, dans lequel la plaque (1118) comprend un réseau d'aimants (832).

3. Système selon la revendication 1, dans lequel la voie de passage indirecte comprend un chemin sinueux (1212, 1228, 1244, 1260) entre l'entrée (1204, 1220, 1236, 1252, 1268, 1284) et la sortie (1208, 1224, 1240, 1256, 1272, 1288).

4. Système selon la revendication 1, dans lequel le sac (1140, 1200, 1216, 1232, 1248, 1264, 1282) comprend des barrières (1246, 1262, 1278, 1294) autour desquelles le courant de fluide s'écoule.

5. Système selon la revendication 2, dans lequel le réseau d'aimants est agencé en un réseau de Halbach (1360).

6. Système selon la revendication 1, dans lequel la texture aide à maintenir les billes magnétiques contre une paroi du sac (1140, 1200, 1216, 1232, 1248, 1264, 1282).

7. Système selon la revendication 1, dans lequel la pompe (716, 736) est configurée pour déplacer un fluide dans le sac (1140, 1200, 1216, 1232, 1248, 1264, 1282) à un débit supérieur à 10 ml/min.

8. Système selon la revendication 1, comprenant en outre une chambre (728) pour concentrer les cellules dans le courant de fluide, dans lequel la sortie du sac est en communication fluidique avec la chambre (728) pour concentrer les cellules dans le courant de fluide après que les billes magnétiques sont enlevées du courant de fluide.

9. Système selon la revendication 8, dans lequel la chambre (728) comprend un orifice supérieur, un orifice inférieur, et un orifice latéral.

10. Système selon la revendication 9, dans lequel le courant de fluide entre dans la chambre par l'intermédiaire de l'orifice latéral.

11. Système selon la revendication 1, dans lequel la plaque (1118) comprend des tenons (1132, 1136) pour attacher le sac (1140, 1200, 1216, 1232, 1248, 1264, 1282).

12. Système selon la revendication 1, dans lequel la plaque (1118) comprend un dispositif de serrage (1128).
